Europäisches Patentamt

European Patent Office

Office européen des brevets

(11) Numéro de publication: **0 585 325 B1**

# FASCICULE DE BREVET EUROPEEN

(45) Date de publication du fascicule du brevet: **06.09.95**  (51) Int. Cl.⁶: **A61K 7/48**, A61K 7/06, A61K 35/78

(21) Numéro de dépôt: **92911286.0**

(22) Date de dépôt: **19.05.92**

(86) Numéro de dépôt internationale :
**PCT/FR92/00444**

(87) Numéro de publication internationale :
**WO 92/20322 (26.11.92 92/29)**

(54) **COMPOSITION COSMETIOUE OU PHARMACEUTIOUE, NOTAMMENT DERMATOLOGIOUE, DESTINEE A FAVORISER LA PIGMENTATION DE LA PEAU OU DES CHEVEUX, CONTENANT UN EXTRAIT DE CYPERUS ET SON PROCEDE DE FABRICATION.**

(30) Priorité: **22.05.91 FR 9106176**

(43) Date de publication de la demande:
**09.03.94 Bulletin 94/10**

(45) Mention de la délivrance du brevet:
**06.09.95 Bulletin 95/36**

(84) Etats contractants désignés:
**BE CH DE ES FR GB GR IT LI**

(56) Documents cités:

Patent Abstracts of Japan, vol. 14, no. 211
(C-715), 2 mai 1990, &
JP,A,2048515(SHISEIDO) 19 février 1990, voir
le résumé

Patent Abstracts of Japan, vol. 12, no. 103
(C-485), 5 avril 1988, & JP,A,62234006 (SHI-
SEIDO) 14 octobre 1987, voir le résumé (citée dans la demande)

(73) Titulaire: **LVMH RECHERCHE**
**25, rue des Peupliers**
**F-92752 Nanterre (FR)**

(72) Inventeur: **MEYBECK, Alain**
**Les Poissons**
**20 ter, rue de Bezons**
**F-92400 Courbevoie (FR)**
Inventeur: **BONTE, Frédéric**
**5, place Charras**
**F-92400 Courbevoie (FR)**
Inventeur: **DUMAS, Marc**
**54, rue de l'Industrie**
**F-92700 Colombes (FR)**

(74) Mandataire: **Portal, Gérard**
**Cabinet Beau de Loménie**
**158, rue de l'Université**
**F-75340 Paris Cédex 07 (FR)**

Rank Xerox (UK) Business Services
(3.10/3.09/3.3.3)

EP 0 585 325 B1

Patent Abstracts of Japan, vol. 12, no. 75 (C-480), 9 mars 1988, & JP,A,62212319 (NIP-PON KAIHATSU CONSULTANT K.K.) 18 septembre 1987, voir le résumé

## Description

La présente invention concerne essentiellement une composition cosmétique ou pharmaceutique, notamment dermatologique, contenant un extrait de Cyperus destinée en particulier à favoriser la pigmentation de la peau ou des cheveux, et son procédé de fabrication.

Elle concerne également l'utilisation d'un extrait de Cyperus pour la préparation d'une telle composition cosmétique ou pharmaceutique.

Les différentes espèces du genre Cyperus font partie de la famille des Cyperacées. On en compte environ 650 dispersées dans l'Ancien et le Nouveau continent, excepté dans les régions froides. Pour déterminer les espèces de ce genre, on peut se reporter à la Flore complète portative, par Gaston Bonnier et G. de Layens, p. 323 et 324. Les Cyperus, appelés également en français Souchet, sont des plantes herbacées annuelles ou pérennes. Elles possèdent généralement des rhizomes. Certaines possèdent également des tubercules. Parmi ces dernières, citons les Cyperus aureus ou Souchet tubéreux, le Cyperus esculentus, le Cyperus olivaris ou Cyperus rotundus L. que l'on trouve notamment en France dans les endroits sablonneux d'une partie du littoral méditerranéen. Les tubercules du Cyperus olivaris, ou rotundus, ont été utilisés contre les maladies de l'estomac, des poumons et de la vessie. Ils entraient dans la composition de diverses préparations pharmaceutiques, telles que l'huile de Scorpion et l'eau thériacale qui ne sont plus employées aujourd'hui.

On sait également par le document JP-A-62-234006 que des extraits de tubercules de Cyperus rotundus présentent une activité anti-inflammatoire et peuvent être utilisés pour la fabrication de compositions cosmétiques ou pharmaceutiques topiques appliquées par voie externe.

On sait par ailleurs par le document US-A-4 908 207 que des extraits de tubercules de la plante Cyperus rotundus L., administrés par voie orale, permettent de traiter les déficiences immunitaires.

On sait encore par le document US-A-4 696 818 que l'on peut réaliser des traitements de cure de désintoxication à l'aide de compositions médicinales contenant notamment du Cyperus rotundus en combinaison avec d'autres extraits de plantes, notamment des racines d'Angelica sinensis.

Encore, on connaît par le document US-A-4 826 684 une composition pharmaceutique, contenant un mélange de terpénoïdes dérivés d'un extrait des tubercules de la plante Cyperus rotondus L., administrée par voie orale pour le traitement des diabètes.

Le document Patent Abstracts of Japan, volume 14, N°211 (C-715), décrit une composition contenant de 0,005 a 10 % en poids sec d'un extrait de rhizomes de Cyperus et une substance tensio-active, cette composition pouvant être formulée sous forme de crème, de gel ou de lotions, pour être appliquée de façon topique sur la peau ou les cheveux.

De même, il est connu par le document Patent Abstracts of Japan, volume 12, N°75 (C-480), une composition pharmaceutique contenant un extrait de rhizomes de Cyperus et des minéraux applicables sur la peau pour le traitement des bleus ou contusions.

La présente invention est basée sur la découverte que les extraits de Cyperus, provenant en particulier des rhizomes ou des tubercules, présentent des propriétés biologiques intéressantes utilisables dans les domaines cosmétique et pharmaceutique. Il a notamment été observé par les inventeurs que ces extraits possédaient de façon inattendue une activité stimulante de la mélanogénèse au niveau des mélanocytes présents dans la peau ou les follicules pileux, et permettaient ainsi de favoriser la pigmentation de la peau ou des cheveux, ainsi que de réaliser un traitement des désordres de la pigmentation de la peau et des cheveux en favorisant plus particulièrement la biosynthèse de mélanine. De très bons résultats dans ce domaine ont été obtenus avec des extraits de tubercules de Cyperus rotundus L.

La présente invention a donc pour but de résoudre le nouveau problème technique consistant en la fourniture d'une nouvelle composition cosmétique ou pharmaceutique, notamment dermatologique, destinée en particulier à favoriser la pigmentation de la peau ou des cheveux.

La présente invention a encore pour but de résoudre le nouveau problème technique consistant en la préparation d'une nouvelle formulation d'une composition cosmétique ou pharmaceutique présentant une bonne activité de stimulation de la mélanogénèse au niveau des mélanocytes présents dans la peau ou dans les follicules pileux.

La présente invention a encore pour but de fournir une solution au nouveau problème technique consistant en la fourniture d'un extrait de plante particulièrement aisé à obtenir, qui présente en lui-même une bonne activité stimulante de la mélanogénèse au niveau des mélanocytes présents dans la peau ou les follicules pileux, sans avoir à réaliser l'isolation d'une substance active quelconque, ces procédés d'isolation étant en général longs et coûteux.

Tous ces nouveaux problèmes techniques sont résolus pour la première fois par la présente invention d'une manière satisfaisante, utilisable à l'échelle industrielle.

Ainsi, selon un premier aspect, la présente invention concerne l'utilisation d'un extrait de Cyperus pour la fabrication d'une composition cosmétique ou pharmaceutique, notamment dermatologique, pour favoriser la pigmentation de la peau ou des cheveux et/ou le traitement des désordres de la pigmentation de la peau et des cheveux, ledit extrait de Cyperus étant éventuellement présent dans un excipient cosmétiquement ou pharmaceutiquement acceptable.

Selon une caractéristique particulière de l'invention, l'extrait de Cyperus est un extrait de rhizomes ou de tubercules. Avantageusement, il s'agit d'un extrait de tubercules de Cyperus rotundus.

Selon une autre caractéristique, on utilise un extrait organique de Cyperus, en particulier de rhizomes ou de tubercules, obtenu avantageusement par un procédé comprenant au moins une étape d'extraction avec un solvant choisi parmi le groupe constitué par le méthanol, l'éthanol ou un mélange hydroalcoolique de ces alcools, l'acétate d'éthyle et le dichlorométhane. D'une façon plus générale, on peut utiliser également comme solvant, des solvants organiques, tels que les hydrocarbures aromatiques, des hydrocarbures aliphatiques ou aromatiques halogénés, des éthers dialcoyliques, des dialcoyl-cétones, des alcanols, des acides carboxyliques et leurs esters ou d'autres solvants, comme le diméthylformamide, le dioxanne, le tétrahydrofuranne et le diméthylsulfoxyde. Parmi les solvants précités, des solvants préférés sont le benzène, le toluène ou le xylène, le chlorure de méthylène, le chloroforme, l'acétate d'éthyle, le méthanol ou l'éthanol. Le rapport de la matière végétale à l'agent d'extraction n'est pas critique, toutefois il est généralement compris entre 1 : 5 et 1 : 20 parties en poids, et il est préférentiellement de 1 : 10 environ. L'extraction s'effectue à des températures comprises entre la température ambiante et le point d'ébullition du solvant utilisé pour l'extraction. Une technique avantageuse d'extraction est la technique dite d'extraction au Soxhlet. Il peut être avantageux et dans certains cas nécessaire d'évaporer le solvant par exemple par lyophilisation et de reprendre les extraits bruts en vue d'une purification. Dans le cadre de la présente invention, l'extraction alcoolique est particulièrement intéressante, notamment en fin de procédure d'obtention de l'extrait en raison du caractère habituellement peu toxique des alcools. Ainsi un alcool particulièrement avantageux est l'éthanol ou le méthanol.

D'une façon générale, la concentration des extraits utilisés conformément à la présente invention, pour la préparation d'une composition cosmétique ou pharmaceutique, exprimée en poids sec est comprise entre 0,002% et 5% en poids, de préférence entre 0,01 % et 1% en poids, par rapport au poids total de la composition.

Les compositions cosmétiques ou pharmaceutiques, notamment dermatologiques, selon la présente invention, peuvent être appliquées par voie topique pour favoriser la pigmentation de la peau et des cheveux, en particulier dans des compositions se présentant sous forme de crèmes, de gels ou de lotions destinées à l'application sur la peau ou les cheveux.

Ainsi, les compositions cosmétiques ou pharmaceutiques, notamment dermatologiques, selon l'invention trouvent diverses applications en cosmétologie ou en dermatologie, en particulier lorsque l'augmentation de la pigmentation est recherchée. Par exemple, ces compositions peuvent être utilisées comme produits solaires pour accélérer ou intensifier le bronzage, ce qui, outre l'avantage esthétique souvent recherché, permet de renforcer les défenses naturelles contre le rayonnement ultraviolet par l'augmentation du taux de mélanine dans l'épiderme. Ces compositions peuvent encore être utilisées, par exemple sous forme de crèmes, pour donner à la peau un aspect plus hâlé, ou encore, sous forme de lotions, pour la prévention et le traitement de l'apparition des cheveux gris. Par ailleurs, en dermatologie, les compositions selon la présente invention peuvent être utilisées comme agents thérapeutiques, seules ou en association avec d'autres médicaments, en particulier en administration topique dans le traitement des disfonctionnements de la mélanogénèse.

Avantageusement, les compositions cosmétiques ou pharmaceutiques selon l'invention, destinées à l'administration topique, contiennent au moins un agent favorisant la pénétration et la diffusion au niveau des structures cutanées concernées tel que ceux utilisés couramment dans les domaines de la cosmétologie et de la dermo-pharmacie comme par exemple, le glycérol, le propylèneglycol, l'acide oléique ou les huiles essentielles, notamment le menthol et l'eucalyptol.

Selon un mode de réalisation avantageux, une composition cosmétique ou pharmaceutique selon l'invention contient en outre une xanthine, en particulier la 1-méthylxanthine, la 3-méthylxanthine, la 3-isobutylméthylxanthine (IBMX) ou la théophylline, de préférence à une concentration pondérale comprise entre 0,001% et 2%, et encore de préférence comprise entre 0,01% et 0,5%, par rapport au poids total de la composition.

Selon un autre mode de réalisation, une composition cosmétique ou pharmaceutique selon l'invention contient en outre de la tyrosine ou l'un de ses dérivés, de préférence à une concentration pondérale comprise entre 0,001% et 10%, par rapport au poids total de la composition.

4

Selon encore un autre mode de réalisation, une composition cosmétique ou pharmaceutique selon l'invention contient en outre au moins une autre substance active, à une concentration efficace, choisie parmi les vitamines, en particulier les vitamines B, la quinine ou ses dérivés, les rubéfiants, tels que le nicotinate de méthyle, un surnageant de culture de fibroblastes de papilles, tel que défini dans le document EP-A-272 920, des hydrolysats de kératine, des oligo-éléments tels que zinc, sélénium, cuivre, des inhibiteurs de 5 $\alpha$-réductase, tels que progestérone, cyprotérone acétate, minoxidil, acide azélaïque et ses dérivés, un 4-méthyl-4-azastéroïde, en particulier la 17-$\beta$-N,N-diéthylcarbamoyl-4-méthyl-4-aza-5-$\alpha$-andros-tan-3-one, ou encore un extrait de Serenoa repens.

Selon encore un autre mode de réalisation, une composition cosmétique ou pharmaceutique selon l'invention contient en outre des phases lamellaires lipidiques hydratées ou des liposomes, incorporant ou non l'extrait de Cyperus précédemment défini.

Enfin, selon un deuxième aspect, la présente invention concerne encore un procédé de traitement cosmétique de la peau ou des cheveux pour favoriser la pigmentation, caractérisé en ce qu'il comprend l'application en une quantité efficace pour réaliser une pigmentation, d'au moins un extrait de Cyperus tel que précédemment défini, incorporé dans un excipient, véhicule ou support cosmétiquement acceptable.

Dans tous les aspects précédents de la présente invention, on peut prévoir la présence de phases lamellaires lipidiques hydratées ou de liposomes, incorporant ou non l'extrait de Cyperus précité.

Le terme "lipidique" dans l'expression "phase lamellaire lipidique" couvre toutes les substances comprenant une chaîne carbonée dite grasse, comportant généralement plus de 5 atomes de carbone, cette substance étant habituellement dénommée "lipide".

Selon l'invention, on utilise à titre de lipide, pour former les phases lamellaires lipidiques, ou les liposomes, des lipides amphiphiles, c'est-à-dire constitués de moléculaires possédant un groupe hydrophile indifféremment ionique ou non ionique et un groupe lipophile, ces lipides amphiphiles étant susceptibles de former des phases lamellaires lipidiques ou des liposomes, en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyol éventuellement polyoxyéthylénés. De telles substances sont par exemple constituées par une lécithine d'oeuf ou de soja, une phosphatidyle sérine, une sphyngomyéline, un cérébroside ou un stéarate de polyglycérol oxyéthyléné.

De préférence, selon l'invention, on utilise un mélange lipidique constitué d'au moins un lipide amphiphile et d'au moins un lipide hydrophobe tel que stérol, comme le cholestérol ou le $\beta$-sitostérol. La quantité, exprimée en mole, de lipide hydrophobe ne doit généralement pas être supérieure à la quantité de lipide amphiphile, et de préférence elle ne doit pas être supérieure à 0,5 fois cette quantité.

L'incorporation en des phases lamellaires lipidiques hydratées ou dans des liposomes, des composés ou des extraits contenant ces composés, utilisés conformément à la présente invention, peut être réalisée selon des techniques de préparation connues, décrites par exemple dans le document EP-B1-0 087 993, éventuellement en combinaison avec le document EP-B1-0 107 559.

D'autres buts, caractéristiques et avantages de l'invention apparaîtront clairement à la lumière de la description explicative qui va suivre faite en référence à plusieurs exemples donnés simplement à titre d'illustration et qui ne saurait donc en aucune façon limiter la portée de l'invention.

Dans les exemples, les pourcentages sont donnés en poids, sauf indication contraire.

EXEMPLE 1

Obtention d'un extrait de Cyperus rotundus L.

On traite au reflux pendant 3 h 500 g de tubercules de Cyperus rotundus L. avec une quantité suffisante de méthanol pour baigner complètement la matière végétale.

On filtre les extraits méthanoliques et on évapore le filtrat méthanolique sous pression réduite, puis on lyophilise. On obtient ainsi l'extrait de tubercules de Cyperus rotundus appelé extrait $I_1$.

EXEMPLE 2

En opérant comme décrit à l'exemple 1, mais en utilisant la plante entière et comme solvant l'acétate d'éthyle, on obtient un extrait de Cyperus rotundus dénommé extrait $I_2$.

EXEMPLE 3

Incorporation d'un extrait de tubercules de Cyperus rotundus dans des phases lamellaires lipidiques hydratées ou dans des liposomes

Un extrait de tubercules de Cyperus rotundus obtenu conformément à l'exemple 1 est incorporé dans des phases lamellaires lipidiques hydratées ou dans des liposomes selon la technique de préparation suivante :

on pèse :
- lécithine de soja : 1,8 g
- $\beta$-sitostérol : 0,2 g
- extrait de Cyperus lyophilisé $I_1$ de l'exemple 1 : 0,03 g

Ces constituants sont dissous dans un mélange constitué de 25 ml de dichlorométhane et 10 ml de méthanol.

On évapore le mélange ainsi obtenu dans un ballon rotatif à température de 60°C sous pression réduite.

Le film lipidique ainsi obtenu est alors repris et dispersé sous agitation dans de l'eau distillée qsp 50 g, à température ambiante pendant 12 h.

La suspension de vésicules lipidiques obtenue est ensuite homogénéisée par un traitement aux ultrasons pendant 10 min à 4°C, sous puissance de 150 W.

La taille moyenne des liposomes ainsi obtenus est d'environ 140 nm.

Selon une variante de réalisation avantageuse, on gélifie ensuite cette suspension en la mélangeant à 50 g de gel de Carbopol® 940 à 1,25% préparé séparément de façon classique. On obtient ainsi 100 g environ d'une suspension gélifiée de liposomes encapsulant l'extrait de Cyperus rotundus, dont la concentration est d'environ 0,030% par rapport au poids total de la suspension.

EXEMPLE 4

Mesure de l'activité d'un extrait de Cyperus rotundus L. selon l'invention sur les mélanocytes en culture

Protocole :

Des mélanocytes murins S91 ensemencés à raison de $2.10^5$ cellules par boîte sont cultivés sur un milieu approprié de manière classique, comprenant du milieu EMEM complémenté, 1 % de sérum de veau foetal et 0,08 $\mu$g de Mitomycine C. 24 h après l'ensemencement, le produit à tester dilué dans du DMSO et incorporé dans du milieu frais est introduit dans la culture.

Après 5 j, on prélève les cellules que l'on isole par centrifugation et on récupère le culot cellulaire que l'on dissout dans de la soude 0,5 N.

On procède à la lecture de la densité optique sur un spectrophotomètre à 405 nm, ce qui permet d'évaluer la quantité de mélanine formée par comparaison à la densité optique d'une solution de mélanine de concentration connue.

On procède également au comptage des cellules, et on calcule le taux de mélanine formée par cellule.

Un extrait de Cyperus rotundus a été testé, à diverses concentrations en $\mu$g/ml, en utilisant comme témoin positif une culture recevant de la ß-MSH (Melanocyte-Stimulating Hormone), à la concentration de $2.10^{-8}$ M.

L'activité A de stimulation de la mélanogénèse par les produits selon l'invention est calculée par la formule suivante :

$$A = \frac{q_p - q_o}{q_t - q_o} \times 100$$

dans laquelle les grandeurs q représentent les quantités de mélanine formée

$q_p$ = culture recevant le produit à tester

$q_t$ = culture recevant la $\beta$MSH

$q_o$ = culture témoin ne recevant aucun produit.

L'activité de l'extrait testé à différentes concentrations, calculée selon la formule ci-dessus, figure au tableau I ci-après.

TABLEAU I

| Extrait de Cyperus rotundus L. (exemple 1) | | | | |
|---|---|---|---|---|
| Concentration produit $\mu$g/ml | nombres de cellules par boîte x $10^3$ | Mélanine $\mu$g pour $10^6$ cellules | Activité % | t |
| Témoin (DMSO aucun produit) | 152 ± 8 | 65 ± 1 | 0 | |
| $\beta$-MSH à $2.10^{-8}$ M | 158 ± 5 | 149 ± 3 | 100 | |
| 2,5 | 147 ± 3 | 71 ± 4 | + 8 | NS |
| 10 | 148 ± 7 | 84 ± 0 | + 25 | S |
| 25 | 147 ± 7 | 94 ± 3 | + 38 | S |
| 50 | 155 ± 8 | 96 ± 3 | + 41 | S |

Il résulte à partir du tableau I ci-dessus que l'extrait de Cyperus selon l'invention stimule la production de mélanine dans une proportion significative, ce qui représente un résultat tout à fait inattendu pour un homme de l'art.

On donnera ci-après divers exemples de formulation de composition cosmétique ou pharmaceutique, notamment dermatologique.

EXEMPLE 5

Gel bronzant pour le visage

| | |
|---|---|
| Extrait de Cyperus (poids sec) selon l'exemple 1 | 0,1 g |
| Ethanol | 40,- g |
| Eau distillée | 20,- g |
| Gel Carbopol® 940 à 1% | qsp 100 g |

EXEMPLE 6

Crème solaire bronzante

| | |
|---|---|
| Extrait de Cyperus (poids sec) selon l'exemple 1 | 0,02 g |
| Stéarate d'isocétyle | 8,- g |
| Huile d'arachide hydrogénée | 10,- g |
| Huile de lanoline | 3,5 g |
| Alcool cétylique | 5,- g |
| Alcool stéarylique | 2,5 g |
| Huile de vaseline légère | 10,- g |
| Monoester phosphorique de l'alcool cétylique OE neutralisé | 3,- g |
| Octylméthoxy cinnamate | 5,- g |

Cette phase est émulsionnée avec une phase aqueuse qsp 100 g contenant :

| | |
|---|---|
| Pantothénol | 0,1 g |
| Conservateurs | 0,2 g |

EXEMPLE 7

Lotion pour renforcer la protection solaire naturelle

| Alcool | 42,5 g |
|---|---|
| Propylèneglycol | 3,- g |
| Menthol | 0,05 g |
| Hydroxypropylméthylcellulose | 1,5 g |
| Extrait de Cyperus selon l'exemple 1 | 0,03 g |
| Excipients aqueux parfumés | qsp 100 g |

Cette lotion est appliquée localement, de préférence deux fois par jour, quotidiennement pendant 3 à 8 jours précédant les expositions prolongées au soleil.

Les applications quotidiennes peuvent être poursuivies lors de la période d'exposition.

EXEMPLE 8

Lotion tonique capillaire contre les cheveux gris

| Extrait de Cyperus selon l'exemple 2 | 0,01 g |
|---|---|
| 3-méthylxanthine | 0,03 g |
| Alcool | 30,- g |
| Excipients aqueux parfumés avec solubilisant de parfum | qsp 100 g |

Cette lotion peut être appliquée sur les cheveux et le cuir chevelu deux fois par jour par cures de trois mois.

EXEMPLE 9

Lotion tonique capillaire contre les cheveux gris

| Extrait de Cyperus rotundus selon l'exemple 1 | 1,0 g |
|---|---|
| Propylèneglycol | 5,0 g |
| Solubilisant de parfum (Crémophor RH 40®) | 0,5 g |
| Ethanol | 35,0 g |
| Excipients aqueux parfumés qsp | 100,0 g |

Cette lotion peut être appliquée sur les cheveux et le cuir chevelu deux fois par jour pour un traitement d'attaque destiné à diminuer rapidement l'apparition des cheveux blancs.

EXEMPLE 10

Gel dermatologique destiné à favoriser la pigmentation de la peau

| Extrait de Cyperus selon l'exemple 1 | 0,1 g |
|---|---|
| Ethanol | 35,0 g |
| Eau distillée | 15,0 g |
| Excipient gélifiant, par exemple Gel de Carbopol 940® à 1,25 % | qsp 100 g |

Ce gel est utilisé 1 à 2 fois par jour en application locale sur les zones de la peau à traiter.

EXEMPLE 11

Lotion capillaire contre les cheveux gris

| | |
|---|---|
| Extrait de Cyperus selon l'exemple 1 | 0,03 g |
| L-tyrosine éthyl ester, HCl | 1,0 g |
| Ethanol | 40,0 g |
| Excipient aqueux parfumé avec solubilisant de parfum | qsp 100 g |

Cette lotion, appliquée sur les cheveux et le cuir chevelu quotidiennement, permet de retarder l'apparition de cheveux gris.

EXEMPLE 12

Gel bronzant

| | |
|---|---|
| Extrait de Cyperus selon l'exemple 1 | 0,02 g |
| Filtre solaire (EUSOLEX 232 TS®) | 8,0 g |
| Ethanol | 40,0 g |
| Excipient gélifiant (comprenant : Carbopol 940® à 1,25 %) | qsp 100 g |

EXEMPLE 13

Lotion prévenant l'apparition des cheveux gris

| | |
|---|---|
| Suspension de liposomes encapsulant un extrait de Cyperus, selon l'exemple 3 | 50 g |
| Carbopol 940® | 0,05 g |
| Tyrosinate de glucose | 0,05 g |
| Complexe d'oligo-élément | 0,1 g |
| Théophylline | 0,01 g |
| Conservateur | 0,05 g |
| Excipient aqueux, par exemple eau distillée | qsp 100 ml |

On applique cette solution le soir sur le cuir chevelu au niveau des zones grisonnantes par cure de 4 mois.

EXEMPLE 14

Lotion pour le corps

| | |
|---|---|
| Extrait de Cyperus rotundus selon l'exemple 1 | 0,01 g |
| N-malyltyrosine | 1,00 g |
| Propylèneglycol | 3,00 g |
| Excipients aqueux parfumés avec solubilisant | qsp 100,00 g |

## Revendications

1. Utilisation d'un extrait de Cyperus pour la fabrication d'une composition cosmétique ou pharmaceutique, notamment dermatologique, pour favoriser la pigmentation de la peau ou des cheveux et/ou le traitement des désordres de la pigmentation de la peau et des cheveux, ledit extrait de Cyperus étant éventuellement présent dans un excipient cosmétiquement ou pharmaceutiquement acceptable.

2. Utilisation selon la revendication 1, caractérisée en ce qu'il s'agit d'un extrait de rhizomes ou de tubercules en particulier un extrait de tubercules de Cyperus rotondus L.

3. Utilisation selon la revendication 1 ou 2, caractérisée en ce que l'extrait de Cyperus précité est un extrait organique de Cyperus, en particulier de rhizomes ou de tubercules de Cyperus, de préférence obtenu par un procédé comprenant au moins une étape d'extraction avec un solvant choisi parmi le groupe constitué par le méthanol, l'éthanol ou un mélange hydroalcoolique de ces alcools, le dichlorométhane et l'acétate d'éthyle.

4. Utilisation selon l'une des revendications 1 à 3, caractérisée en ce que la concentration en extrait de Cyperus précité, exprimé en poids sec, est comprise entre 0,002 % et 5 % en poids, de préférence entre 0,01 % et 1 % en poids, par rapport au poids total de la composition.

5. Utilisation selon l'une des revendications précédentes, caractérisée en ce que la composition contient en outre une xanthine en particulier la 1-méthylxanthine, la 3-méthylxanthine, la 3-isobutylméthylxanthine (IBMX) ou la théophylline, de préférence à une concentration pondérale comprise entre 0,001 et 2 %, et encore de préférence comprise entre 0,01 % et 0,5 %, par rapport au poids total de la composition.

6. Utilisation selon l'une des revendications précédentes, caractérisée en ce que la composition contient en outre de la tyrosine ou l'un de ses dérivés, de préférence à une concentration pondérale comprise entre 0,001 % et 10 %, par rapport au poids total de la composition.

7. Utilisation selon l'une des revendications précédentes, caractérisée en ce que la composition contient en outre au moins une autre substance active, à une concentration efficace, choisie parmi les vitamines, en particulier les vitamines B, la quinine ou ses dérivés, les rubéfiants, tels que le nicotinate de méthyle, un surnageant de culture de fibroblastes de papilles, des hydrolysats de kératine, des oligo-éléments tels que zinc, sélénium, cuivre, des inhibiteurs de 5 $\alpha$-réductase, tels que progestérone, cyprotérone acétate, minoxidil, acide azélaïque et ses dérivés, un 4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

8. Utilisation selon l'une des revendications précédentes, caractérisée en ce que la composition contient des phases lamellaires lipidiques hydratées ou des liposomes incorporant ou non l'extrait de Cyperus précité.

9. Utilisation selon l'une des revendications précédentes, caractérisée en ce qu'on formule la composition sous une forme permettant une application topique, en particulier sous forme de crème, de gel ou de lotion destiné à l'application sur la peau ou sur les cheveux.

10. Procédé de traitement cosmétique de pigmentation de la peau ou des cheveux, pour favoriser la pigmentation, caractérisé en ce qu'il comprend l'application en une quantité cosmétiquement efficace pour réaliser une pigmentation, d'au moins un extrait de Cyperus tel que précédemment défini, incorporé dans un excipient, véhicule ou support cosmétiquement acceptable.

## Claims

1. Use of an extract of Cyperus for the preparation of a cosmetic or pharmaceutical composition, especially dermatological composition, intended for promoting the pigmentation of the skin or hair, and/or the treatment of disorders of the pigmentation of the skin and hair, said extract of Cyperus being optionally present in a cosmetically or pharmaceutically acceptable excipient.

2. Use according to claim 1, characterized in that it is an extract of rhizomes or tubers, in particular an extract of tubers of Cyperus rotundus L.

3. Use according to claim 1 or 2, characterized in that the above-mentioned extract of Cyperus is an organic extract of Cyperus, in particular of rhizomes or tubers of Cyperus, preferably obtained by a process comprising at least one extraction step with a solvent selected from the group consisting of methanol, ethanol or an aqueous-alcoholic mixture of these alcohols, dichloromethane and ethyl acetate.

4. Use according to one of claims 1 to 3, characterized in that the concentration of above-mentioned extract of Cyperus, expressed by dry weight, is between 0.002% and 5% by weight, preferably between 0.01% and 1% by weight, based on the total weight of the composition.

5. Use according to one of the preceding claims, characterized in that the composition also contains a xanthine, in particular 1-methylxanthine, 3-methylxanthine, 3-isobutymethylxanthine (IBMX) or theophylline, preferably at a concentration by weight of between 0.001 and 2% and particularly preferably of between 0.01% and 0.5%, based on the total weight of the composition.

6. Use according to one of the preceding claims, characterized in that the composition also contains tyrosine or one of its derivatives, preferably at a concentration by weight of between 0.001% and 10%, based on the total weight of the composition.

7. Use according to one of the preceding claims, characterized in that the composition also contains an effective concentration of at least one other active substance selected from vitamins, in particular the B vitamins, quinine or its derivatives, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone acetate, minoxidil, azelaic acid and its derivatives, a 4-methyl-4-azasteroid, in particular 17-$\beta$-N,N-diethylcarbamoyl-4-methyl-4-aza-5-$\alpha$-androstan-3-one, or else an extract of Serenoa repens.

8. Use according to one of the preceding claims, characterized in that the composition contains hydrated lipidic lamellar phases or liposomes, which may or may not incorporate the above-mentioned extract of Cyperus.

9. Use according to one of the preceding claims, characterized in that the composition is formulated in a form suitable for topical application, in particular in the form of a cream, gel or lotion intended for application to the skin or to the hair.

10. Process for the cosmetic treatment of pigmentation of the skin or hair, for promoting the pigmentation, characterized in that it comprises the application in a cosmetically efficient quantity for producing a pigmentation of at least one extract of Cyperus such as defined above, incorporated into a cosmetically or pharmaceutically acceptable excipient, vehicle, or carrier.

**Patentansprüche**

1. Verwendung eines Cyperus-Extrakts zur Herstellung einer kosmetischen oder pharmazeutischen, insbesondere dermatologischen Zusammensetzung, zur Förderung der Pigmentation der Haut oder der Haare und/oder zur Behandlung von Störungen der Pigmentation der Haut und der Haare, welcher Cyperus-Extrakt gegebenenfalls in einem kosmetisch oder pharmazeutisch akzeptablen Träger vorliegt.

2. Verwendung nach Anspruch 1, dadurch gekennzeichnet, daß es sich um einen Rhizom- oder Knollenextrakt handelt, insbesondere um einen Extrakt der Knollen von Cyperus rotundus L.

3. Verwendung nach Anspruch 1 oder 2, dadurch gekennzeichnet, daß der vorgenannte Cyperus-Extrakt ein organischer Cyperus-Extrakt ist, insbesondere von den Rhizomen oder Knollen des Cyperus, welcher vorzugsweise mittels eines Verfahrens erhalten wurde, das mindestens einen Extraktionsschritt mit einem Lösungsmittel ausgewählt aus der Gruppe bestehend aus Methanol, Ethanol oder einer wässerigen alkoholischen Mischung dieser Alkohole, Dichlormethan und Ethylacetat umfaßt.

4. Verwendung nach einem der Ansprüche 1 bis 3, dadurch gekennzeichnet, daß die Konzentration des vorgenannten Cyperus-Extrakts, als Trockengewicht ausgedrückt, zwischen 0,002 und 5 Gew.-%, vorzugsweise zwischen 0,01 und 1 Gew.-%, bezogen auf das Gesamtgewicht der Zusammensetzung, liegt.

5. Verwendung nach einem der vorhergehenden Ansprüche, dadurch gekennzeichnet, daß die Zusammensetzung außerdem ein Xanthin, insbesondere 1-Methylxanthin, 3-Methylxanthin, 3-Isobutylmethylxanthin (IBMX) oder Theophyllin, vorzugsweise in einer Gewichtskonzentration zwischen 0,001 und 2%, und insbesondere zwischen 0,01 und 0,5%, bezogen auf das Gesamtgewicht der Zusammensetzung, enthält.

6. Verwendung nach einem der vorhergehenden Ansprüche, dadurch gekennzeichnet, daß die Zusammensetzung außerdem Tyrosin oder eines seiner Derivate, vorzugsweise in einer Gewichtskonzentration zwischen 0,001% und 10%, bezogen auf das Gesamtgewicht der Zusammensetzung, umfaßt.

7. Verwendung nach einem der vorhergehenden Ansprüche, dadurch gekennzeichnet, daß die Zusammensetzung außerdem mindestens eine andere aktive Substanz in einer wirksamen Konzentration enthält, ausgewählt aus Vitaminen, insbesondere B-Vitaminen, Chinin oder dessen Derivaten, Reizmitteln, wie Methylnikotinat, einen Papillenfibroblasten-Kulturüberstand, Keratinhydrolysate, Spurenelemente wie Zink, Selen, Kupfer, Inhibitoren von 5-$\alpha$-Reductase, wie Progesteron, Cyproteronacetat, Minoxidil, Azelainsäure und deren Derivate, ein 4-Methyl-4-azasteroid, insbesondere 17-$\beta$-N,N-Diethyl-carbamoyl-4-methyl-4-aza-5-$\alpha$-androstan-3-on, oder noch einen Extrakt von Serenoa repens, enthält.

8. Verwendung nach einem der vorhergehenden Ansprüche, dadurch gekennzeichnet, daß die Zusammensetzung hydratisierte lamellenartige Lipidphasen oder Liposome, gegebenenfalls in den vorerwähnten Cyperus-Extrakt inkorporiert, enthält.

9. Verwendung nach einem der vorhergehenden Ansprüche, dadurch gekennzeichnet, daß die Zusammensetzung in einer Form, die eine topische Anwendung ermöglicht, insbesondere in Form einer Creme, eines Gels oder einer Lotion zur Anwendung auf die Haut oder auf die Haare zubereitet ist.

10. Verfahren zur kosmetischen Behandlung der Pigmentation der Haut oder der Haare, zur Verbesserung der Pigmentation, dadurch gekennzeichnet, daß es das Aufbringen einer kosmetisch wirksamen Menge zur Realisierung einer Pigmentation mindestens eines Cyperus-Extrakts, wie voranstehend definiert, inkorporiert in ein kosmetisch akzeptables Träger-, Vehikel- oder Hilfsmittel, umfaßt.